# EUROPÄISCHE PATENTSCHRIFT

(11) **EP 0 512 502 B1**
(45) Veröffentlichungstag und Bekanntmachung des Hinweises auf die Patenterteilung: **26.03.1997**
(21) Anmeldenummer: 92107633.7
(22) Anmeldetag: 06.05.1992
(51) Int. Cl.: C07C 17/38, C07C 19/08

(54) **Verfahren zur Entfernung olefinischer Verunreinigungen aus 1,1,1,2,3,3,3 -Heptafluorpropan**
Process for removal of olefinic impurities from 1,1,1,2,3,3,3-heptafluoropropane
Procédé pour l'élimination d'impuretés oléfiniques de 1,1,1,2,3,3,3-heptafluoropropane

(30) Priorität: 08.05.1991 DE 4115025
(43) Veröffentlichungstag der Anmeldung: 11.11.1992
(73) Patentinhaber: SOLVAY (Société Anonyme), B-1050 Bruxelles (BE)
(72) Erfinder: Hopp, Peter, Dr., W-6238 Hofheim am Taunus (DE); Jansen, Rolf-Michael, Dr., W-6233 Kelkheim (Taunus) (DE)
(74) Vertreter: Jacques, Philippe

(56) Entgegenhaltungen:
- EP-A- 0 370 688
- GB-A- 902 590
- GB-A- 1 031 409
- US-A- 2 409 274
- CHEMICAL ABSTRACTS, vol. 71, no. 13, 29. September 1969, Columbus, Ohio, US; abstract no. 60600w, R. FONTANELLI ET AL. 'Fluoroolefins. IV. Base-catalyzed reactions of fluoropropylenes with alcohols. Stereochemical aspects of the reactions' Seite 366 ;

## Beschreibung

Die Erfindung betrifft ein Verfahren zur Entfernung olefinischer Verunreinigungen aus 1,1,1,2,3,3,3-Heptafluorpropan (R227), dessen Herstellung aus der GB-PS 902 590 bekannt ist. Diese Verbindung ist als Ersatzstoff für die ozongefährdenden vollhalogenierten Fluorchlorkohlenwasserstoffe vorgesehen. Um R 227 im Bereich der Kältetechnik oder als Treibmittel für Pharma-Aerosole verwenden zu können, müssen störende und giftige olefinische Verunreinigungen, die bei der Synthese - zum Teil nur im Spurenbereich - entstehen, vollständig entfernt werden. Dies ist mit herkömmlichen physikalischen Methoden wie Destillation oder Adsorption in einem wirtschaftlich vertretbaren Rahmen nicht möglich. Deshalb muß nach einer Möglichkeit gesucht werden, die störenden Verunreinigungen durch Reaktion mit geeigneten Substanzen in nicht-toxische Verbindungen umzuwandeln oder in solche, die auf physikalischem Wege ohne großen Aufwand von R 227 abgetrennt werden können.

Es hat sich nun als günstig herausgestellt, die bei der Herstellung von R 227 entstehenden Olefine - wie 2-H-Pentafluorpropen - mit Alkoholen in Gegenwart von Basen umzusetzen. Hierbei entstehen ausschließlich hochsiedende Verbindungen, die bei der Reindestillation von R 227 leicht abgetrennt werden können. Außerdem wird R 227 nicht angegriffen, und die Reaktion läuft schnell und quantitativ ab.

Gemäß der 1966 veröffentlichten GB-A-1 031 409 wird 2,2,2-Trifluor-1-chlor-1-bromethan mittels Alkoholen und Basen von Fluorbutenen befreit. Nach der EP-A-0 370 688 aus 1990 werden dagegen ganz allgemein gesättigte Fluorkohlenwasserstoffe mittels festen Metalloxiden von Fluorolefinen befreit.

Es ist zwar aus Chemical Abstracts 1971, Vol. 13: 60600w bekannt, daß man aus 2-H-Pentafluorpropen und Alkoholen in Gegenwart von KOH ein Gemisch von höhersiedenden Ethern und Estern herstellen kann. Die Umsätze sind jedoch nicht quantitativ. Daher war nicht zu erwarten, daß man Spuren von 2-H-Pentafluorpropen und anderen Olefinen, gelöst in R 227, durch Umsetzung mit Alkoholen quantitativ entfernen kann. Außerdem war zu befürchten, daß R 227 analog zu vielen anderen Fluorkohlenwasserstoffen mit Alkoholen Azeotrope bildet.

Gegenstand der Erfindung ist ein Verfahren zur Entfernung olefinischer Verunreinigungen der allgemeinen Formel CₙHₘFₚCl_{q}, wobei n = 2 - 6, m = 0 - 8, p = 1 - 12, q = 0 - 8 und m + p + q = 2n ist, aus 1,1,1,2,3,3,3-Heptafluorpropan (R 227), das dadurch gekennzeichnet ist, daß man das verunreinigte R 227 mit einem Alkohol und einer Base, deren pK-Wert bei 8 bis 14 liegt, bei Temperaturen von -20 bis 100°C und Drücken von 1 bis 50 bar in Kontakt bringt und gleichzeitig oder anschließend das R 227 abdestilliert.

Bei den Verunreinigungen handelt es sich vor allem um 1,1,3,3,3-Pentafluorpropen (2-H-Pentafluorpropen) und Perfluorpropen.

Vorzugsweise verwendet man einen einwertigen Alkohol der allgemeinen Formel CₐH₂ₐ₊₁OH, CₐH₂ₐ(OH)₂, CₐH₂ₐ₋₁OH oder CₐH₂ₐ₋₂(OH)₂, mit a = 1 bis 6, insbesondere mit a = 1 bis 3. Besonders geeignet sind Methanol, Ethanol, i-Propanol und Ethylenglykol (Glykol).

Als Basen verwendet man solche, deren pK-Wert bei 8 bis 14 liegt, insbesondere NaOH, KOH oder Na-Phenolat.

Die Temperatur liegt vorzugsweise bei 0 bis 50°C, und der Druck beträgt vorzugsweise 1 bis 10 bar. Bezogen auf R 227 beträgt die Menge an Alkohol vorzugsweise 0,5 bis 10 Gew.-% und die Menge an Base vorzugsweise 0,01 bis 5 Gew.-%.

Die Erfindung soll anhand der folgenden Beispiele näher erläutert werden.

### Beispiel 1 bis 5:

Ein beheizbarer Rührautoklav (V = 300 ml) wurde mit 100 g 1,1,1,2,3,3,3-Heptafluorpropan (R 227), das ca. 500 ppm 2-H-Pentafluorpropen (PFP) enthielt, sowie mit 10 g eines Alkohols und 1 g einer Base beschickt. Anschließend wurde der Autoklav auf 50°C erwärmt (Druck 9.3 bar) und 4 Stunden bei dieser Temperatur gerührt. Der Gehalt an PFP wurde gaschromatographisch verfolgt. Die eingesetzten Alkohole und Basen und die Ergebnisse sind in Tabelle 1 dargestellt.

**Tabelle 1**

| Alkohol | Base | Gehalt PFP [ppm] nach 4 Stunden |
|---|---|---|
| Methanol | NaOH | u.N.* |
| Methanol | KOH | u.N.* |
| Ethanol | NaOH | u.N.* |
| i-Propanol | NaOH | 30 |
| Glykol | NaOH | u.N.* |

| | | |
|---|---|---|
| * u.N. = unter Nachweisgrenze (<1 ppm) | | |

### Beispiel 6:

Ein Destillationsgefäß wurde mit 80 kg R 227, das ca. 200 ppm 2-H-Pentafluorpropen enthielt, sowie 600 g Methanol und 15 g KOH beschickt. Anschließend wurde das Gefäß auf 30°C erwärmt und 12 Stunden bei dieser Temperatur gerührt. Der Gehalt an PFP war auf unter 10 ppm gefallen.

## Patentansprüche

1. Verfahren zur Entfernung olefinischer Verunreinigungen der allgemeinen Formel CₙHₘFₚCl_{q}, wobei n = 2 - 6, m = 0 - 8, p = 1 - 12, q = 0 - 8 und m + p + q = 2n ist, aus 1,1,1,2,3,3,3-Heptafluorpropan (R 227), dadurch gekennzeichnet, daß man das verunreinigte R 227 mit einem Alkohol und einer Base, deren pk-Wert bei 8 bis 14 liegt, bei Temperaturen von -20 bis 100°C und Drücken von 1 bis 50 bar in Kontakt bringt und gleichzeitig oder anschließend das R 227 abdestilliert.

2. Verfahren nach Anspruch 1, dadurch gekennzeichnet, daß man einen Alkohol der allgemeinen Formel CₙH₂ₙ₊₁OH, CₙH₂ₙ(OH)₂, CₙH₂ₙ₋₁OH oder CₙH₂ₙ₋₂(OH)₂ mit n = 1 - 6, vorzugsweise n = 1 - 3 verwendet.

3. Verfahren nach einem der Ansprüche 1 bis 2, dadurch gekennzeichnet, daß man bei 0 bis 50°C und 1 bis 10 bar arbeitet.

4. Verfahren nach einem der Ansprüche 1 bis 3, dadurch gekennzeichnet, daß man als Alkohol Ethylenglykol, Methanol, Ethanol oder 1-Propanol verwendet.

5. Verfahren nach einem der Ansprüche 1 bis 4, dadurch gekennzeichnet, daß man als Base NaOH, KOH oder Na-Phenolat verwendet.

6. Verfahren nach einem der Ansprüche 1 bis 5, dadurch gekennzeichnet, daß man 0,5 bis 10 Gew.-% Alkohol und 0,01 bis 5 Gew.-% Base verwendet, bezogen auf R 227.

## Claims

1. A process for the removal of olefinic impurities of the formula CₙHₘFₚCl_{q} wherein n = 2 - 6, m = 0 - 8, p = 1 - 12, q = 0 - 8 and m + p + q = 2n, from 1,1,1,2,3,3,3-heptafluoropropane (R 227), which comprises bringing the contaminated R 227 into contact with an alcohol and a base whose pK is from 8 to 14 at temperatures of from -20 to 100°C and at pressures of from 1 to 50 bar, and simultaneously or subsequently removing the R 227 by distillation.

2. The process as claimed in claim 1, wherein an alcohol of the formula CₙH₂ₙ₊₁OH, CₙH₂ₙ(OH)₂, CₙH₂ₙ₋₁OH or CₙH₂ₙ₋₂(OH)₂ where n = 1 - 6, preferably 1 - 3, is used.

3. The process as claimed in Claim 1 or 2, wherein the process is carried out at from 0 to 50°C and at from 1 to 10 bar.

4. The process as claimed in any one of claims 1 to 3, wherein the alcohol used is ethylene glycol, methanol, ethanol or i-propanol.

5. The process as claimed in any one of claims 1 to 4, wherein the base used is NaOH, KOH or sodium phenoxide.

6. The process as claimed in any one of claims 1 to 5, wherein from 0.5 to 10% by weight of alcohol and from 0.01 to 5% by weight of base are used, based on R 227.

## Revendications

1. Procédé pour éliminer, à partir du 1,1,1,2,3,3,3-heptafluoropropane (R 227), des impuretés oléfiniques de formule générale CₙHₘFₚCl_{q}, où n = 2-6, m = 0-8, p = 1-12, q = 0-8 et m+p+q = 2n, caractérisé en ce qu'on met en contact le R 227 contaminé avec un alcool et une base dont le pK est compris entre 8 et 14, à des températures de -20 à 100°C et sous des pressions de 1 à 50 bar, et simultanément ou ensuite, on soutire le R 227 par distillation.

2. Procédé selon la revendication 1, caractérisé en ce qu'on utilise un alcool de formule générale CₐH₂ₐ₊₁OH, CₐH₂ₐ(OH)₂, CₐH₂ₐ₋₁OH ou CₐH₂ₐ₋₂(OH)₂, avec n = 1-6, de préférence n = 1-3.

3. Procédé selon l'une des revendications 1 à 2, caractérisé en ce qu'on travaille à une température de 0 à 50°C et sous une pression de 1 à 10 bar.

4. Procédé selon l'une des revendications 1 à 3, caractérisé en ce qu'on utilise comme alcool l'éthylèneglycol, le méthanol, l'éthanol ou l'isopropanol.

5. Procédé selon l'une des revendications 1 à 4, caractérisé en ce qu'on utilise comme base du NaOH, du KOH ou du phénolate de Na.

6. Procédé selon l'une des revendications 1 à 5, caractérisé en ce qu'on utilise de 0,5 à 10 % en poids d'alcool et de 0,01 à 5 % en poids de la base, par rapport au R 227.
